# EUROPEAN PATENT APPLICATION

(11) **EP 4 495 100 A1**
(43) Date of publication of application: **22.01.2025**
(21) Application number: 23771012.4
(22) Date of filing: 06.03.2023
(51) Int. Cl.: C07C 67/08, C07C 67/54, C07C 67/62, C07C 69/708

(54) **METHOD FOR SEPARATING WATER FROM MIXTURE OF PGME, PGMEA, AND WATER**

(30) Priority: 14.03.2022 KR 20220031500
(71) Applicant: Benit M Co., Ltd., Ulsan 44602 (KR)
(72) Inventor: KANG, Ki Joon, Ulju-gun, Ulsan 44992 (KR); HARVIANTO, Gregorius Rionugroho, Ulju-gun, Ulsan 44992 (KR); KIM, Kwang Hyun, Ulju-gun, Ulsan 44992 (KR)
(74) Representative: Ipside
(86) International application number: PCT/KR2023/003026
(87) International publication number: WO 2023/177133

(57) **Abstract**

There is provided a method of removing water from a mixture of PGME, PGMEA, and water. The method includes a reactor introduction step of introducing PGME and acetic acid into a reactor, a distillation column introduction step of introducing a liquid in the reactor into a distillation column, an entrainer introduction step of introducing an entrainer into an upper portion of the distillation column, and a condensation step of condensing, in a condenser, a mixture of water and the entrainer, which evaporates from the upper portion of the distillation column.

## Description

### Technical Field

The disclosure relates to a method of removing water from a mixture of PGME, PGMEA, and water, and more particularly, to a method of removing water from a mixture of PGME, PGMEA, and water, so that a reaction rate for the production of PGMEA may increase and simultaneously the purity of PGMEA may increase by removing, from a reactant and a reaction product, water produced as a by-product in the production of PGMEA used as an environmentally friendly solvent.

### Background Art

Propylene glycol monomethyl ether acetate (PGMEA) is an environmentally friendly solvent used for various purposes, such as a paint solvent and an electronic material solvent.

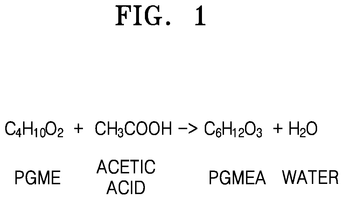

Referring to FIG. 1, PGMEA is produced by reacting propylene glycol monomethyl ether (PGME) and acetic acid in the presence of a catalyst, and water is produced as a by-product according to the reaction.

When PGMEA is produced, water produced by a reaction should be removed in order not to decrease a reaction rate, and water removal is also necessary to increase the purity of the produced PGMEA.

The boiling temperature of a reactant and a reaction product under the atmospheric pressure is about 100 °C for water, about 118 °C for acetic acid, about 120 °C for PGME, and about 146 °C for PGMEA. In this regard, since the boiling temperature of water is the lowest, which is about 100 °C, it seems that water may be removed by evaporating water through distillation, but water-PGME and water-PGMEA azeotrope at about 96 °C to about 98 °C under the atmospheric pressure.

In other words, water-PGME and water-PGMEA evaporate together by azeotroping with water at a temperature lower than the boiling temperature of water. Since PGME, which is a reactant, and water evaporate and are discharged in a weight ratio of about 52:48 by such a reaction, there is a problem in that PGME necessary for the reaction should be additionally supplemented, and there is a problem in that the produced PGMEA is also azeotropically discharged with water in a weight ratio of about 56:44, and thus a reaction yield is reduced.

In order to solve the above-described problems, in the related art, toluene which form azeotropes with water at a temperature lower than the azeotropic temperature of water-PGME and water-PGMEA is used as an entrainer to remove water.

However, when toluene is used as an entrainer, water-toluene has an azeotropic point of about 84.3 °C under the atmospheric pressure, but a ternary azeotropic point of water-PGME-toluene under the atmospheric pressure is formed at about 83.9 °C which is lower than the azeotropic point of water-toluene. Accordingly, there is a problem in that PGME and water first azeotrope with toluene in a weight ratio of about 0.76:1, and since PGME is a water-soluble solvent, there is a problem in that a large amount of PGME which is a reactant is lost to wastewater.

In addition to a method of using toluene as an entrainer, in the related art, a method of using cyclohexane as an entrainer is used to remove water.

However, when cyclohexane is used as an entrainer, cyclohexane-water has an azeotropic point of about 69.5 °C under the atmospheric pressure, but a ternary azeotropic point of water-PGME-cyclohexane under the atmospheric pressure is formed at about 69 °C which is lower than the azeotropic point of cyclohexane-water. Accordingly, there is a problem in that PGME and water first azeotrope with cyclohexane in a weight ratio of about 1:1, and since PGME is a water-soluble solvent, there is still a problem in that a large amount of PGME, which is a reactant, is lost to wastewater.

In addition, there are methods using various entrainers in the related art, but since the proposed entrainers also azeotrope with PGME or PGMEA, there is a problem in that there is no choice but to accept the loss of PGME or PGMEA. (U.S. Patent Publication No. US 4,544,453)

In the related art, a method of recovering PGME from water by using ethyl benzyl ether as an extractant is used, but since PGME, which is water-soluble, is not completely extracted from water, there is a problem in that loss of PGME occurs.

In addition, there is also a method using pervaporation to recover PGME or PGMEA mixed with water, but this method requires a pervaporation membrane with a large membrane area, and thus, it is not suitable for commercial application.

### Disclosure

### Technical Problem

The disclosure is to solve the above-described problems, and more particularly, relates to a method of removing water from a mixture of PGME, PGMEA, and water, so that a reaction rate for the production of PGMEA may increase and simultaneously the purity of PGMEA may increase by removing, from a reactant and a reaction product, water produced as a by product in the production of PGMEA used as an environmentally friendly solvent.

### Technical Solution

A method of removing water from a mixture of PGME, PGMEA, and water includes a reactor introduction step of introducing PGME and acetic acid into a reactor, a distillation column introduction step of introducing a liquid in the reactor into a distillation column, an entrainer introduction step of introducing an entrainer into an upper portion of the distillation column, and a condensation step of condensing, in a condenser, a mixture of water and the entrainer, which evaporates from the upper portion of the distillation column.

The entrainer may include at least one of isopropyl acetate, ethyl acetate, and n-propyl acetate.

The method may further include a reflux step of separating a liquid phase condensed in the condenser into an organic material layer and a water layer and refluxing the organic material layer to the distillation column, and a discharge step of separating a liquid phase condensed in the condenser into an organic material layer and a water layer and discharging the water layer.

A catalyst to promote a reaction between the PGME and the acetic acid may be mixed with the liquid introduced into the reactor and introduced into the distillation column.

The catalyst may include a material having a boiling temperature of 220 °C or higher under the atmospheric pressure or having a boiling temperature of 140 °C or higher at 20 mmHg.

A solid catalyst to promote a reaction between the PGME and the acetic acid may be filled in the distillation column, and the solid catalyst may be filled under an inflow point where the liquid in the reactor is introduced into the distillation column.

The method may further include a re-introduction step of re-introducing the liquid discharged through a lower portion of the distillation column into the reactor.

The number of separation stages of the distillation column may be 21 or more.

An inflow point where the liquid in the reactor is introduced into the distillation column may be a point apart from the upper portion of the distillation column by 8 separation stages, or may be lower than the point apart from the upper portion of the distillation column by 8 separation stages.

An inflow point where the liquid in the reactor is introduced into the distillation column may be a point apart from a lower portion of the distillation column by 2 separation stages, or may be higher than the point apart from the lower portion of the distillation column by 2 separation stages.

The reactor and the distillation column may be integrally formed.

A temperature of the upper portion of the distillation column may be greater than or equal to an azeotropic point of the mixture of the entrainer and the water, and a temperature of a lower portion of the distillation column may be a boiling point of the mixture discharged through the lower portion of the distillation column.

When the entrainer is isopropyl acetate, the pressure of the upper portion of the distillation column may be -0.925 kg/cm²G to 0.5 kg/cm²G, when the entrainer is ethyl acetate, a pressure of the upper portion of the distillation column may be -0.89 kg/cm²G to 0.5 kg/cm²G, and when the entrainer is n-propyl acetate, a pressure of the upper portion of the distillation column may be -0.96 kg/cm2G to 0.5 kg/cm2G.

An inflow point where the liquid in the reactor is introduced into the distillation column may be 90 °C to 140 °C when the pressure of the upper portion of the distillation column is the atmospheric pressure.

The distillation column may include at least one of a tray, random packing, and structured packing.

### Advantageous Effects

The disclosure relates to a method of removing water from a mixture of PGME, PGMEA, and water, and has an advantage in that a reaction rate for the production of PGMEA can be increased by removing, from a reactant and a reaction product, water produced as a by-product in the production of PGMEA used as an environmentally friendly solvent.

In addition, the disclosure has an advantage in that the purity of PGMEA can be increased by removing, from a reactant and a reaction product, water produced as a by-product in the production of PGMEA used as an environmentally friendly solvent.

Furthermore, the disclosure has an advantage in that the loss of a reactant and a reaction product can be minimized by removing, from the reactant and the reaction product, water produced as a by-product in the production of PGMEA used as an environmentally friendly solvent, and accordingly, PGMEA which is an environmentally friendly solvent used as a paint solvent, an electronic material solvent, or the like can be economically produced.

### Description of Drawings

FIG. 1 shows a reaction formula for producing propylene glycol monomethyl ether acetate (PGMEA) by reacting propylene glycol monomethyl ether (PGME) and acetic acid in the presence of a catalyst.
FIG. 2 is a flowchart of a method of removing water from a mixture of PGME, PGMEA, and water, according to an embodiment.
FIGS. 3 and 4 are diagrams showing a reactor and a distillation column to remove water from a mixture of PGME, PGMEA, and water, according to embodiments.
FIGS. 5 and 6 are diagrams showing that a reactor and a distillation column to remove water from a mixture of PGME, PGMEA, and water are integrally formed, according to embodiments.
FIG. 7 is a composition distribution inside a distillation column when isopropyl acetate was used as an entrainer, according to an embodiment.
FIG. 8 is a composition distribution inside a distillation column when ethyl acetate was used as an entrainer, according to another embodiment.
FIG. 9 is a composition distribution inside a distillation column when n-propyl acetate was used as an entrainer, according to another embodiment.
FIG. 10 is a composition distribution inside a distillation column when toluene was used as an entrainer.

### Mode for Invention

This specification clarifies the scope of the disclosure, describes the principles of the disclosure, and discloses embodiments so that those of ordinary skill in the art to which the disclosure pertains can practice the disclosure. The disclosed embodiments may be implemented in various forms.

The expressions "include" or "may include" used in various embodiments of the disclosure refer to the presence of a corresponding function, operation, or element of the disclosure, and are not to be construed as limiting one or more additional function, operation, or element. In addition, it should be understood that, in various embodiments of the disclosure, the terms, such as "include" or "have", are intended to designate that a feature, number, step, operation, element, component, or combination thereof described in the specification is present, and do not preclude in advance the possibility of the presence or addition of other features, operations, elements, components, or combinations thereof.

It should be further understood that, when it is described that an element is "connected" or "coupled" to another element, the element may be directly connected or coupled to the other element, but another new element may be present between the element and the other element. On the contrary, it should be understood that, when it is described that an element is "directly connected" or "directly coupled" to another element, another new element is not present between the element and the other element.

While such terms as "first", "second", etc., may be used to describe various elements, such elements must not be limited to the above terms. The above terms are used only to distinguish one element from another element. The temperature according to an embodiment may be a degree Celsius (°C).

The disclosure relates to a method of removing water from a mixture of propylene glycol monomethyl ether (PGME), propylene glycol monomethyl ether acetate (PGMEA), and water, and relates to a method of removing water from a mixture of PGME, PGMEA, and water, which may increase a reaction rate for the production of PGMEA and simultaneously increase the purity of PGMEA by removing, from a reactant and a reaction product, water produced as a by-product in the production of PGMEA used as an environmentally friendly solvent. Hereinafter, embodiments of the disclosure will be described in detail with reference to the accompanying drawings.

A method of removing water from a mixture of PGME, PGMEA, and water according to an embodiment is to prevent loss of acetic acid, PGME, and PGMEA by azeotroping acetic acid, PGME, and PGMEA at a temperature lower than the azeotropic temperature of water and an entrainer by using the entrainer that azeotropes with water but does not azeotrope with acetic acid, PGME, and PGMEA at a temperature lower than the azeotropic temperature of water and the entrainer.

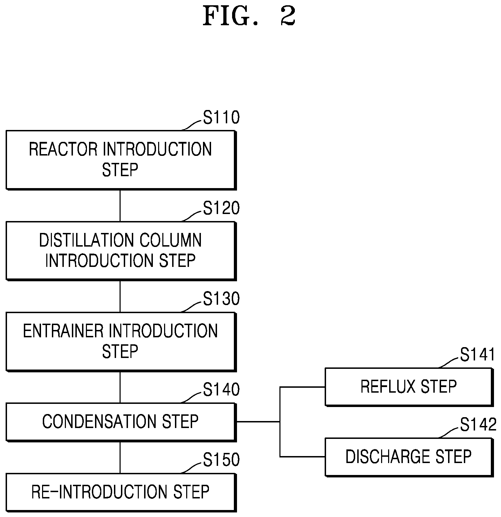

Referring to FIG. 2, a method of removing water from a mixture of PGME, PGMEA, and water according to an embodiment includes a reactor introduction step (S110), a distillation column introduction step (S120), an entrainer introduction step (S130), and a condensation step (S140).

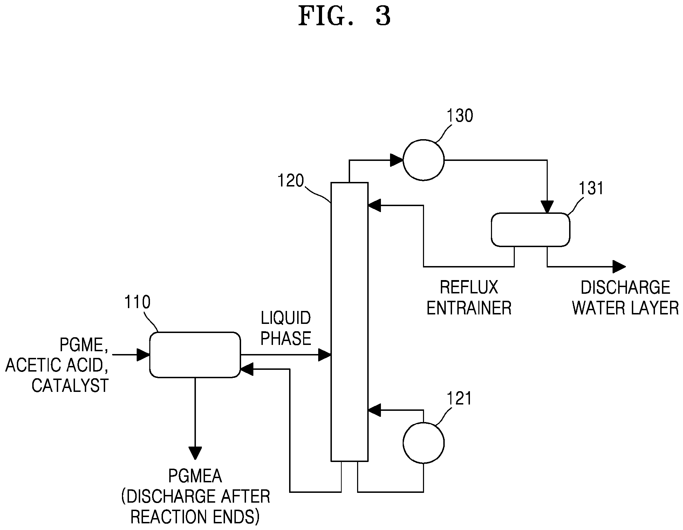

Referring to FIGS. 2 and 3, in the reactor introduction step (S110), PGME and acetic acid are introduced into a reactor 110. According to an embodiment, PGMEA may be produced by reacting PGME and acetic acid in the presence of a catalyst, as shown in FIG. 1. In this state, water may be produced as a by-product.

In the distillation column introduction step (S120), a liquid in the reactor 110 is introduced into a distillation column 120. The distillation column 120 separates substances by vaporizing a liquid, and as the liquid in the reactor 110 is introduced into the distillation column 120, water may be removed from a mixture of PGME, PGMEA, and water.

In the entrainer introduction step (S130), an entrainer is introduced into an upper portion of the distillation column 120. The entrainer may be used to remove water from the mixture of PGME, PGMEA, and water by introducing the liquid in the reactor 110 into the distillation column 120.

In the condensation step (S140), a mixture of water and the entrainer, which evaporates from the upper portion of the distillation column 120, is condensed in a condenser 130. The mixture of the water and the entrainer, which evaporates from the upper portion of the distillation column 120, may be condensed in the condenser 130 and then moved to an oil-water separator 131.

According to an embodiment, the entrainer may include at least one of isopropyl acetate, ethyl acetate, and n-propyl acetate.

Since isopropyl acetate azeotropes with water at about 77 °C in a weight ratio of about 8.1:1 under the atmospheric pressure and does not azeotrope with acetic acid, PGME, and PGMEA and also does not form a ternary azeotrope with water, isopropyl acetate may be used as an entrainer.

Since ethyl acetate azeotropes with water at about 72 °C in a weight ratio of about 10:1 under the atmospheric pressure and does not azeotrope with acetic acid, PGME, and PGMEA and also does not form a ternary azeotrope with water, ethyl acetate may be used as an entrainer.

Since n-propyl acetate azeotropes with water at about 83 °C in a weight ratio of about 5.3:1 under the atmospheric pressure and does not azeotrope with acetic acid, PGME, and PGMEA and also does not form a ternary azeotrope with water, n-propyl acetate may be used as an entrainer.

However, ethyl acetate, isopropyl acetate, and n-propyl acetate cause a transesterification reaction of PGME in the presence of a catalyst. Therefore, in order to suppress a transesterification reaction and simultaneously remove water from a mixture of PGME, PGMEA, and water, PGME and an entrainer should not be mixed in the presence of a catalyst through the distillation column 120.

To this end, the number of separation stages of the distillation column 120 according to an embodiment may be 21 or more, and an inflow point where the liquid in the reactor 110 is introduced into the distillation column 120 may be a point apart from the upper portion of the distillation column 120 by 8 stages or may be lower than the point apart from the upper portion of the distillation column 120 by 8 stages.

When the number of separation stages of the distillation column 120 is less than 21, acetic acid, PGME, and PGMEA may be easily lost, and as ethyl acetate or isopropyl acetate, which is an entrainer, is leaked through a lower portion of the distillation column 120, ethyl acetate or isopropyl acetate is in contact with PGME in the presence of a catalyst, thereby causing a side reaction and impurities.

Therefore, the number of separation stages of the distillation column 120 may be 21 or more to prevent acetic acid, PGME, and PGMEA from being lost through the upper portion of the distillation column 120 or prevent ethyl acetate or isopropyl acetate, which is an entrainer, from being leaked through the lower portion of the distillation column 120 and contacting with PGME in the presence of a catalyst.

However, when the number of separation stages of the distillation column 120 is too large, the effect may not be large compared to the cost for the configuration of the distillation column 120. Therefore, the number of separation stages of the distillation column 120 may be 120 or less.

In this regard, as the distillation column 120 is separated by a tray installed inside the distillation column 120, the number of separation stages may be formed, and thus, the number of separation stages of the distillation column 120 may be determined by the number of trays.

A separation stage of the distillation column 120 may be an actual stage constituting the distillation column 120, not a theoretical stage. In addition, the degree of separation of substances in the distillation column 120 is related to the number of separation stages, tray spacing is only related to processing capacity, and thus, a tray spacing may be 300 mm to 950 nm, which is a common tray spacing used in practice.

In general, in order to process the same capacity, when a tray spacing is small, a diameter of a distillation column becomes larger compared to a case where a tray spacing is large, and a tray may generally have a thickness of 2 mm to 6 mm.

However, since the thickness of a tray may be determined by the mechanical strength of the tray in consideration of a diameter of a distillation column, the thickness of a tray may not affect the separation of substances in a distillation column. Therefore, the thickness of a tray is not limited to 2 mm to 6 mm, and the thickness of a tray may vary.

According to an embodiment, the distillation column 120 may include at least one of a tray, random packing, and structured packing. A separation stage of the distillation column 120 may include a tray, such as a bubble cap tray, a perforated tray, or a valve tray, and may include one of structured packing and random packing.

However, the disclosure is not limited thereto, and a separation stage of the distillation column 120 according to an embodiment may include a combination of a tray, random packing, structured packing, and the like.

When the distillation column 120 includes the structured packing or the random packing, the number of stages of the structured packing or the random packing may be converted into the number of trays, and when the number of stages of the structured packing or the random packing is converted into the number of trays, the number of separation stages of the distillation column 120 may be at least 21 and not more than 120.

A method of converting the number of stages of the structured packing or the random packing into the number of trays may utilize, for calculation, height equivalent to a theoretical plate (HETP) data presented by manufacturers or literature, such as Perry handbook, Distillation Design (Kister), and Ludwig's Applied Process Design for Chemical and Petrochemical Plants.

According to an embodiment, structured packing is characterized by surface area per unit volume, and structured packing used in the distillation column 120 may have a surface area of 125 m²/m³ to 1,000 m²/m³. In this case, a packed bed corresponding to one tray has a height of about 100 mm to about 800mm.

In other words, in a case where the distillation column 120 includes structured packing having a surface area per unit volume of 1,000 m²/m³, when a packed bed has a height of 2.1 m, 21 separation stages may be formed. Alternatively, when the distillation column 120 includes structured packing having a surface area per unit volume of 1,000 m²/m³ and a height of 1 m and 11 trays, 21 separation stages may be formed.

In this manner, the number of stages of the structured packing may be converted into the number of trays, and a method of converting the number of the structured packing or the random packing into the number of trays is a well-known art, and thus, detailed description thereof is omitted.

According to an embodiment, an inflow point where the liquid in the reactor 110 is introduced into the distillation column 120 may be a point apart from the upper portion of the distillation column 120 by 8 separation stages, or may be lower than the point apart from the upper portion of the distillation column 120 by 8 separation stages.

When an inflow point where the liquid in the reactor 110 is introduced into the distillation column 120 is not apart from the upper portion of the distillation column 120 by 8 separation stages and is higher than a point apart from the upper portion of the distillation column 120 by 8 separation stages, ethyl acetate, isopropyl acetate, or n-propyl acetate, which is an entrainer, is mixed with PGME in the presence of a catalyst and may cause transesterification of PGME.

Specifically, as a distance between a point where the liquid in the reactor 110 is introduced and an entrainer to be introduced into the upper portion of the distillation column 120 decreases, the entrainer may be mixed with PGME in the presence of a catalyst, thereby causing a transesterification reaction of PGME, and acetic acid, PGME, and PGMEA may be lost through the upper portion of the distillation column 120.

Therefore, an inflow point where the liquid in the reactor 110 is introduced into the distillation column 120 may be a point apart from the upper portion of the distillation column 120 by 8 separation stages, or may be lower than the point apart from the upper portion of the distillation column 120 by 8 separation stages.

According to an embodiment, when the distillation column 120 includes 21 stages, an inflow point where the liquid in the reactor 110 is introduced into the distillation column 120 may be an 8^{th} stage apart from the upper portion of the distillation column 120 by 8 separation stages or may be lower than the 8^{th} stage.

In this regard, an entrainer to be introduced to the upper portion of the distillation column 120 may be introduced through the uppermost stage of the distillation column 120 or a stage directly below the uppermost stage. However, the disclosure is not limited thereto.

In addition, according to an embodiment, an inflow point where the liquid in the reactor 110 is introduced into the distillation column 120 may be a point apart from the lower portion of the distillation column 120 by 2 separation stages, or may be higher than the point apart from the lower portion of the distillation column 120 by 2 separation stages.

However, the disclosure is not limited thereto, and when the number of separation stages included in the distillation column 120 is large, an inflow point where the liquid in the reactor 110 is introduced into the distillation column 120 may be changed as necessary.

The method of removing water from the mixture of PGME, PGMEA, and water according to an embodiment may further include a reflux step (S141) and a discharge step (S142).

Referring to FIGS. 2 and 3, in the reflux step (S141), a liquid phase condensed in the condenser 130 is separated into an organic material layer and a water layer in the oil-water separator 131, and the organic material layer is refluxed to the distillation column 120. In the discharge step (S142), a liquid phase condensed in the condenser 130 is separated into an organic material layer and a water layer, and the water layer is discharged.

The oil-water separator 131 is capable of separating an organic material layer and a water layer, and a liquid phase condensed in the condenser 130 may be separated into an organic material layer and a water layer through the oil-water separator 131.

The liquid phase condensed in the condenser 130 is a mixture of an entrainer (ethyl acetate, isopropyl acetate, or n-propyl acetate) and water, and the organic material layer separated by the oil-water separator 131 may include an entrainer.

In the reflux step (S141), the organic material layer separated through the oil-water separator 131 is refluxed to the distillation column 120, and an entrainer included in the organic material layer may be re-introduced into the distillation column 120 through the reflux step (S141).

In the discharge step (S142), the water layer separated through the oil-water separator 131 is discharged, and the water layer separated through the oil-water separator 131 may be discharged to the outside in the discharge step (S142).

However, the disclosure is not limited thereto, and part of water discharged from the water layer in the oil-water separator 131 may be refluxed to the distillation column 120 to suppress acetic acid, PGME, and PGMEA from being discharged through the upper portion of the distillation column 120.

In this regard, the organic material layer including an entrainer, which is refluxed to the distillation column 120, or the water layer may be refluxed to the top of the distillation column 120. However, considering the ease of operation of the distillation column 120, the organic material layer or the water layer may be partially refluxed to the middle of the distillation column 120.

However, even in this case, a reflux point of the organic material layer should be higher than an inflow point where the liquid in the reactor 110 is introduced into the distillation column 120 to prevent contact between PGME and the entrainer in the presence of a catalyst.

The method of removing water from the mixture of PGME, PGMEA, and water according to an embodiment may further include a re-introduction step (S150).

Referring to FIGS. 2 and 3, in the re-introduction step (S150), the liquid discharged through the lower portion of the distillation column 120 is re-introduced into the reactor 110. When water is removed through the distillation column 120, water may be removed from the liquid discharged through the lower portion of the distillation column 120.

In the re-introduction step (S150), the liquid at the lower portion of the distillation column 120, from which water is removed, is re-introduced into the reactor 110. The liquid at the lower portion of the distillation column 120 may be acetic acid, PGME, PGMEA, or a catalyst. The liquid at the lower portion of the distillation column 120 continues to circulate through the reactor 110 and the distillation column 120, and in this process, PGME and acetic acid react to be almost converted into PGMEA. After the reaction ends, PGMEA may be discharged from the reactor 110.

In the re-introduction step (S150), after the liquid at the lower portion of the distillation column 120 is re-introduced into the reactor 110, while the liquid at the lower portion of the distillation column 120 continues to circulate through the reactor 110 and the distillation column 120, a reaction in which PGMEA and water are produced by acetic acid, PGME, and a catalyst may continue, and water produced in the reaction may be removed in the distillation column 120.

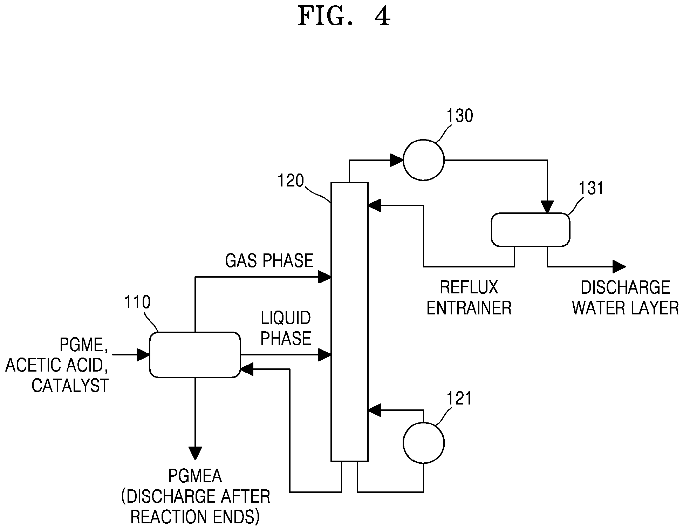

Referring to FIG. 4, some gas may be generated in the reactor 110 due to an increase in temperature of the reactor 110, and the gas generated in the reactor 110 may be introduced into the distillation column 120.

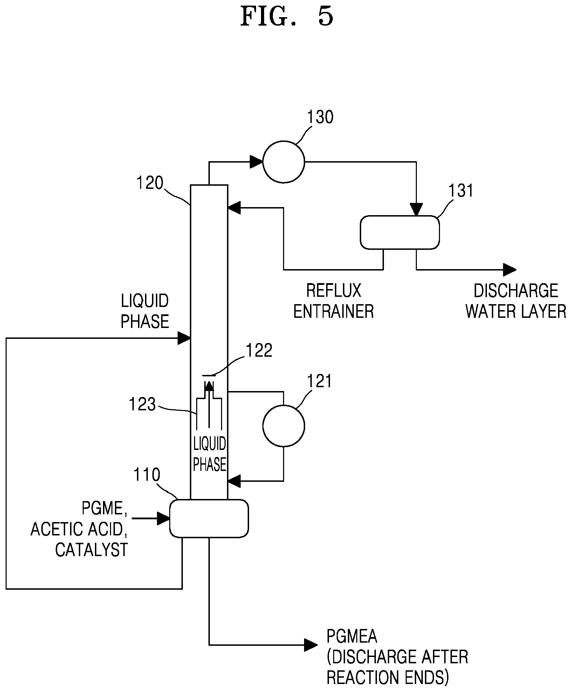

Referring to FIG. 5, the reactor 110 and the distillation column 120 according to an embodiment may be integrally formed. While the reactor 110 is installed at the lower portion of the distillation column 120, the reactor 110 and the distillation column 120 may be integrally formed.

When the reactor 110 and the distillation column 120 are integrally formed, an installation space of a device may be reduced, and as the liquid at the lower portion of the distillation column 120 is re-introduced into the reactor 110 through a downcomer 123 of a collector tray 122 installed at the lower portion of the distillation column 120, a separate liquid transfer device is not necessary.

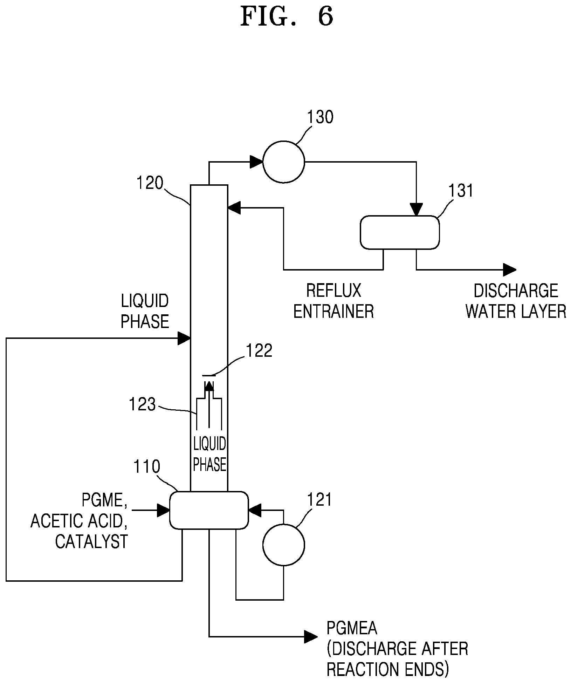

In this state, a reboiler 121 may be installed in the distillation column 120, and a liquid may be re-heated through the reboiler 121. In addition, referring to FIG. 6, the reboiler 121 may be installed in the reactor 110.

According to an embodiment, a catalyst to promote a reaction between the PGME and the acetic acid may be mixed with the liquid introduced into the reactor 110 and introduced into the distillation column 120.

The catalyst introduced into the reactor 110 may include a material having a boiling temperature of 220 °C or higher under the atmospheric pressure or a boiling temperature of 140 °C or higher at 20 mmHg. Specifically, the catalyst may include a material having a boiling temperature of 220 °C or higher sufficiently greater than 146 °C which is a boiling temperature of PGMEA under the atmospheric pressure.

When the boiling temperature of the catalyst is lower than 220 °C, the catalyst may boil and rise above an inflow point of the distillation column 120, and accordingly, may be mixed with an entrainer, thereby causing a transesterification reaction of PGME. Therefore, the catalyst introduced into the reactor 110 may include a material having a boiling temperature of 220 °C or higher under the atmospheric pressure.

In this regard, in a case where a boiling point of the catalyst is measured, when the temperature is raised to 220 °C under the atmospheric pressure, the catalyst may decompose such that the boiling temperature cannot be measured. Therefore, the boiling temperature of the catalyst may be measured under a temperature condition at which the catalyst does not decompose, and accordingly, the catalyst may include a material having a boiling temperature of 140 °C or higher at 20 mmHg.

According to an embodiment, a solid catalyst to promote a reaction between the PGME and the acetic acid may be filled in the distillation column 120, and the solid catalyst may be filled under an inflow point where the liquid in the reactor 110 is introduced into the distillation column 120.

According to an embodiment, the catalyst may be a solid catalyst, and the catalyst may be a solid catalyst that is not dissolved by a reactant. When the catalyst is a solid catalyst that is not dissolved by a reactant, the solid catalyst may be filled under an inflow point where the liquid in the reactor 110 is introduced into the distillation column 120.

As the solid catalyst is filled under the inflow point where the liquid in the reactor 110 is introduced into the distillation column 120, the distillation column 120 may serve as the reactor 110 and the distillation column 120 simultaneously.

Even in this case, conditions for preventing an entrainer from being in contact with PGME in the presence of a catalyst are the same as those described above, and operating conditions are also the same as those described below. A method of filling a solid catalyst in the distillation column 120 may include filling the solid catalyst in a section separate from a separation stage, or filling the solid catalyst by attaching the solid catalyst to a tray, random packing, structured packing, or the like.

However, the disclosure is not limited thereto, the catalyst may also be introduced into the reactor 110, and the catalyst may be introduced into and filled in at least one of the reactor 110 and the distillation column 120.

According to an embodiment, the temperature of the upper portion of the distillation column 120 may be greater than or equal to an azeotropic point of a mixture of the entrainer and the water, and the temperature of the lower portion of the distillation column 120 may be a boiling point of the mixture discharged through the lower portion of the distillation column 120.

Only when the temperature of the upper portion of the distillation column 120 is greater than or equal to an azeotropic point of a mixture of the entrainer and the water, the mixture of the entrainer and the water may evaporate to remove water. Therefore, the temperature of the upper portion of the distillation column 120 may be determined through an azeotropic point of a mixture of the entrainer and the water. In addition, the temperature of the lower portion of the distillation column 120 may be a boiling point of a mixture (PGMEA, catalyst, or the like) discharged through the lower portion of the distillation column 120.

According to an embodiment, when the entrainer is isopropyl acetate, the pressure of the upper portion of the distillation column 120 may be -0.925 kg/cm²G to 0.5 kg/cm²G, when the entrainer is ethyl acetate, the pressure of the upper portion of the distillation column 120 may be -0.89 kg/cm²G to 0.5 kg/cm²G, and when the entrainer is n-propyl acetate, the pressure of the upper portion of the distillation column 120 may be -0.96 kg/cm²G to 0.5 kg/cm²G.

When the entrainer is isopropyl acetate and the pressure of the upper portion of the distillation column 120 is -0.925 kg/cm²G to 0.5 kg/cm²G, the temperature of the upper portion of the distillation column 120 may be adjusted to about 25 °C to about 88 °C, and the temperature of the lower portion of the distillation column 120 may be adjusted to about 82 °C to about 160 °C.

In this regard, when the pressure of the upper portion of the distillation column 120 is greater than 0.5 kg/cm²G, the temperature of the lower portion of the distillation column 120 increases to 160 °C or higher, and thus, impurities may be generated by decomposition of PGMEA.

In addition, when the pressure of the upper portion of the distillation column 120 is less than -0.925 kg/cm²G, the gas temperature of the upper portion of the distillation column 120 decreases to 25 °C or lower, and thus, a refrigerator may be required to condense a gas, which is an uneconomical process configuration. Therefore, when the entrainer is isopropyl acetate, the pressure of the upper portion of the distillation column 120 may be -0.925 kg/cm²G to 0.5 kg/cm²G.

When the entrainer is ethyl acetate and the pressure of the upper portion of the distillation column 120 is -0.89 kg/cm²G to 0.5 kg/cm²G, the temperature of the upper portion of the distillation column 120 may be adjusted to about 25 °C to about 83 °C, and the temperature of the lower portion of the distillation column 120 may be adjusted to about 88 °C to about 160 °C.

In this regard, when the pressure of the upper portion of the distillation column 120 is greater than 0.5 kg/cm²G, the temperature of the lower portion of the distillation column 120 increases to 160 °C or higher, and thus, impurities may be generated by decomposition of PGMEA.

In addition, when the pressure of the upper portion of the distillation column 120 is less than -0.89 kg/cm²G, the gas temperature of the upper portion of the distillation column 120 decreases to 25 °C or lower, and thus, a refrigerator may be required to condense a gas, which is an uneconomical process configuration. Therefore, when the entrainer is ethyl acetate, the pressure of the upper portion of the distillation column 120 may be -0.89 kg/cm²G to 0.5 kg/cm²G.

When the entrainer is n-propyl acetate and the pressure of the upper portion of the distillation column 120 is -0.96 kg/cm²G to 0.5 kg/cm²G, the temperature of the upper portion of the distillation column 120 may be adjusted to about 25 °C to about 95 °C, and the temperature of the lower portion of the distillation column 120 may be adjusted to about 73 °C to about 160 °C.

In this regard, when the pressure of the upper portion of the distillation column 120 is greater than 0.5 kg/cm²G, the temperature of the lower portion of the distillation column 120 increases to 160 °C or higher, and thus, impurities may be generated by decomposition of PGMEA.

In addition, when the pressure of the upper portion of the distillation column 120 is less than -0.96 kg/cm²G, the gas temperature of the upper portion of the distillation column 120 decreases to 25 °C or lower, and thus, a refrigerator may be required to condense a gas, which is an uneconomical process configuration. Therefore, when the entrainer is n-propyl acetate, the pressure of the upper portion of the distillation column 120 may be -0.96 kg/cm²G to 0.5 kg/cm²G.

According to an embodiment, the temperature of an inflow point where the liquid in the reactor 110 is introduced into the distillation column 120 may be 90 °C to 140 °C when the pressure of the upper portion of the distillation column 120 is the atmospheric pressure, and may be 65 °C to 115 °C when the pressure of the upper portion of the distillation column 120 is -0.625 kg/cm²G.

When the temperature of the inflow point where the liquid in the reactor 110 is introduced into the distillation column 120 is too low, an entrainer introduced into the upper portion of the distillation column 120 reaches to the inflow point, and thus, the entrainer is in contact with PGME in the presence of a catalyst, thereby causing a transesterification reaction.

In addition, when the temperature of the inflow point where the liquid in the reactor 110 is introduced into the distillation column 120 is too high, a catalyst introduced from the reactor 110 may go up above the inflow point, and thus, an entrainer is in contact with PGME in the presence of the catalyst, thereby causing a transesterification reaction.

In other words, when the temperature of an inflow point where the liquid in the reactor 110 is introduced to the distillation column 120 is too low or high, an entrainer introduced to the upper portion of the distillation column 120 goes down, or a catalyst introduced through the reactor 110 goes up to the upper portion of the distillation column 120, and thus, the entrainer may be in contact with PGME in the presence of the catalyst, thereby causing a transesterification reaction.

Therefore, the temperature of an inflow point where the liquid in the reactor 110 is introduced into the distillation column 120 may be 90 °C to 140 °C when the pressure of the upper portion of the distillation column 120 is the atmospheric pressure, and may be 65 °C to 115 °C when the pressure of the upper portion of the distillation column 120 is -0.625 kg/cm²G.

However, according to an embodiment, the temperature of an inflow point where the liquid in the reactor 110 is introduced into the distillation column 120 may vary depending on the pressure of the upper portion of the distillation column 120, and may vary depending on the pressure of the inflow point, which is determined by the pressure drop inside the distillation column 120, and the composition of a liquid formed at the inflow point.

Through the method of removing water from the mixture of PGME, PGMEA, and water according to an embodiment, acetic acid and PGME, which are reactants, and PGMEA, which is a reaction product, may not be lost through water, and reaction rate may be improved by continuously removing water produced during a reaction while a transesterification reaction of PGME is suppressed.

In addition, as the number of separation stages of the distillation column 120 is set to 21, an inflow point where the liquid in the reactor 110 is introduced into the distillation column 120 is a point apart from the upper portion of the distillation column 120 by 8 separation stages or is formed lower than the point apart from the upper portion of the distillation column 120 by 8 separation stages, and thus, the concentration of an entrainer in a liquid phase at the inflow point where the liquid in the reactor 110 is introduced may be maintained to be less than or equal to 100 ppm by weight.

In addition, as the number of separation stages of the distillation column 120 is set to 21, an inflow point where the liquid in the reactor 110 is introduced into the distillation column 120 is a point apart from the upper portion of the distillation column 120 by 8 separation stages or is formed lower than the point apart from the upper portion of the distillation column 120 by 8 separation stages, and thus, the concentration of an entrainer in the liquid discharged from the lower portion of the distillation column 120 and introduced into the reactor 110 may be maintained to be less than or equal to 10 ppm by weight, and a transesterification reaction of PGME may be minimized.

In the following description, the effects of the disclosure will be described through Examples and Comparative Example of a method of removing water from a mixture of PGME, PGMEA, and water.

### [Example 1]

According to an embodiment, the distillation column 120 included 21 separation stages, and a mixture of water, acetic acid, PGME, PGMEA, and a catalyst was introduced from the reactor 110 into a middle point of the distillation column 120, and simultaneously, isopropyl acetate as an entrainer was introduced into the upper portion of the distillation column 120. P-toluenesulfonic acid (PTSA) having a boiling point of 140 °C at 20 mmHg was used as a catalyst.

The temperature of the upper portion of the distillation column 120 was determined by an azeotropic point of an entrainer and water and the pressure of the upper portion of the distillation column 120, and while the pressure of the upper portion of the distillation column 120 was set to the atmospheric pressure during operation, the temperature of the upper portion of the distillation column 120 was set to about 77 °C.

The temperature of the lower portion of the distillation column 120 was determined by a boiling point of a liquid-phase composition mixture discharged through the lower portion of the distillation column 120 and the pressure of the lower portion of the distillation column 120, and was set to about 135 °C.

The temperature of a certain separation stage above an inflow point where the liquid is introduced from the reactor 110 into the distillation column 120 was adjusted to about 97 °C so that the entrainer did not reach the inflow point, and the catalyst introduced into the inflow point was not mixed with the entrainer.

The temperature of the liquid introduced from the reactor 110 into the distillation column 120 was set to about 60 °C to about 135 °C as the liquid discharged through the lower portion of the distillation column 120 was introduced into the reactor 110 and then re-introduced into the distillation column 120.

In this regard, the temperature of the liquid introduced from the reactor 110 into the distillation column 120 may not be greater than the boiling temperature of the lower portion of the distillation column 120 to prevent large-scale evaporation of the catalyst above the inflow point.

In addition, when the temperature of the liquid introduced from the reactor 110 into the distillation column 120 is lower than 60 °C, energy may be overused by a reboiler, and thus, the temperature of the liquid introduced from the reactor 110 into the distillation column 120 may be greater than 60 °C.

As the reaction proceeds, the composition of the liquid discharged through the lower portion of the distillation column 120 changes, and thus, when the reaction ends, the maximum temperature may become the boiling temperature of the catalyst and PGMEA.

In other words, according to an embodiment, the temperature of the liquid introduced from the reactor 110 into the distillation column 120 may be greater than 60 °C and lower than the temperature of the lower portion of the distillation column 120.

The temperature of a certain separation stage above an inflow point where the liquid is introduced from the reactor 110 into the distillation column 120 may be determined depending on which stage is used as a reference, and the temperature may be between the temperature of the inflow point and the temperature of the top of the distillation column 120. The temperature of the inflow point is about 113 °C, but may be a boiling point at the corresponding pressure of a liquid composition formed at the inflow point, and thus, the temperature of the inflow point may vary as the reaction proceeds.

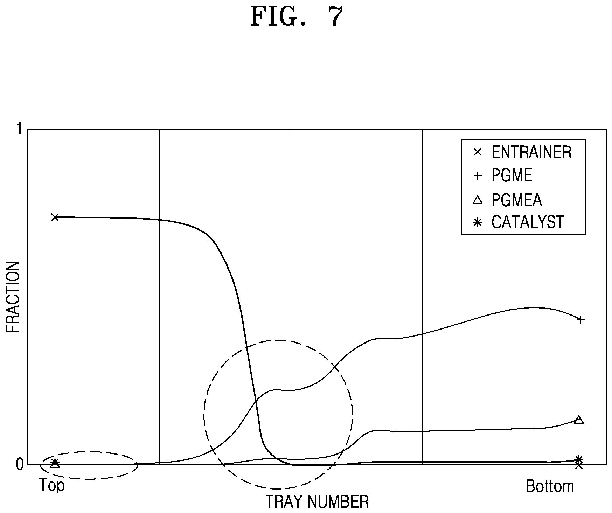

FIG. 7 is a composition distribution inside the distillation column 120 when isopropyl acetate was used as an entrainer. Referring to FIG. 7, through the above-described method, the concentration of acetic acid, PGME, and PGMEA in a gas discharged through the upper portion of the distillation column 120 could be less than or equal to 100 ppm by weight, and leakage of the entrainer through the lower portion of the distillation column 120 could be prevented while the entrainer was not mixed with PGME in the presence of the catalyst in the distillation column 120.

Specifically, referring to FIG. 7, it was found that the entrainer did not leak through the lower portion of the distillation column 120 without leakage of PGME and PGMEA through the upper portion of the distillation column 120, and it was found that there was no section where the entrainer was mixed with PGME in the presence of the catalyst in the distillation column 120. (Referring to FIG. 7, there was no section where the entrainer, the catalyst, and PGME coexisted in the distillation column 120.)

In another embodiment, the pressure of the upper portion of the distillation column 120 may be changed. However, the pressure of the upper portion of the distillation column 120 may be adjusted in a range from -0.925 kg/cm²G to 0.5 kg/cm²G.

In a case where the pressure of the upper portion of the distillation column 120 was -0.625 kg/cm²G during operation, even when the temperature of the upper portion of the distillation column 120 was adjusted to about 53 °C, the temperature of the lower portion of the distillation column 120 was adjusted to about 115 °C, and the temperature of a certain separation stage above the inflow point where the liquid introduced from the reactor 110 into the distillation column 120 was adjusted to 71 °C, the concentration of PGME and PGMEA in the gas discharged through the upper portion of the distillation column 120 was less than or equal to 100 ppm by weight, the entrainer was not mixed with PGME in the presence of the catalyst in the distillation column 120 during operation, and the entrainer did not leak through the lower portion of the distillation column 120.

### [Example 2]

According to another embodiment, the distillation column 120 included 21 separation stages, and a mixture of water, acetic acid, PGME, PGMEA, and a catalyst was introduced from the reactor 110 into a middle point of the distillation column 120, and simultaneously, ethyl acetate as an entrainer was introduced into the upper portion of the distillation column 120. P-toluenesulfonic acid (PTSA) having a boiling point of 140 °C at 20 mmHg was used as a catalyst.

The temperature of the upper portion of the distillation column 120 was determined by an azeotropic point of an entrainer and water and the pressure of the upper portion of the distillation column 120, and while the pressure of the upper portion of the distillation column 120 was set to the atmospheric pressure during operation, the temperature of the upper portion of the distillation column 120 was set to about 72 °C.

The temperature of the lower portion of the distillation column 120 was determined by a boiling point of a liquid-phase composition mixture discharged through the lower portion of the distillation column 120 and the pressure of the lower portion of the distillation column 120, and was set to about 135 °C.

The temperature of a certain separation stage above an inflow point where the liquid was introduced from the reactor 110 into the distillation column 120 was adjusted to about 93 °C so that the entrainer did not reach the inflow point, and the catalyst introduced into the inflow point was not mixed with the entrainer.

The temperature of the liquid introduced from the reactor 110 into the distillation column 120 was set to about 60 °C to about 135 °C as the liquid discharged through the lower portion of the distillation column 120 was introduced into the reactor 110 and then re-introduced into the distillation column 120.

In this regard, the temperature of the liquid introduced from the reactor 110 into the distillation column 120 may not be greater than the boiling temperature of the lower portion of the distillation column 120 to prevent large-scale evaporation of the catalyst above the inflow point.

In addition, when the temperature of the liquid introduced from the reactor 110 into the distillation column 120 is lower than 60 °C, energy may be overused by a reboiler, and thus, the temperature of the liquid introduced from the reactor 110 into the distillation column 120 may be greater than 60 °C.

As the reaction proceeds, the composition of the liquid discharged through the lower portion of the distillation column 120 changes, and thus, when the reaction ends, the maximum temperature may become the boiling temperature of the catalyst and PGMEA.

In other words, according to an embodiment, the temperature of the liquid introduced from the reactor 110 into the distillation column 120 may be greater than 60 °C and lower than the temperature of the lower portion of the distillation column 120.

The temperature of a certain separation stage above an inflow point where the liquid is introduced from the reactor 110 into the distillation column 120 may be determined depending on which stage is used as a reference, and the temperature may be between the temperature of the inflow point and the temperature of the top of the distillation column 120. The temperature of the inflow point is about 108 °C, but may be a boiling point at the corresponding pressure of a liquid composition formed at the inflow point, and thus, the temperature of the inflow point may vary as the reaction proceeds.

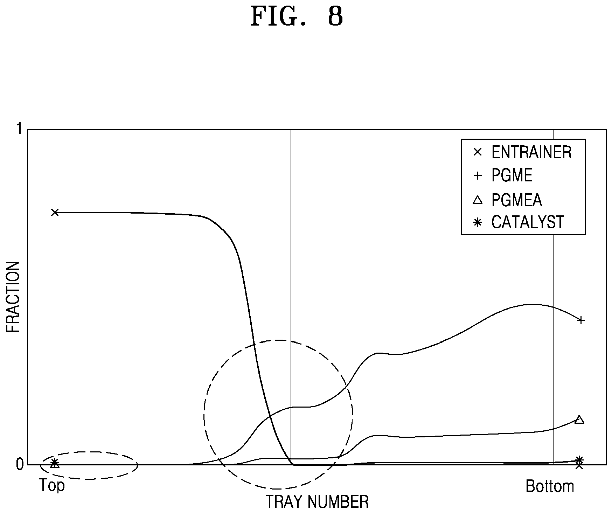

FIG. 8 is a composition distribution inside the distillation column 120 when ethyl acetate was used as an entrainer. Referring to FIG. 8, through the above-described method, the concentration of acetic acid, PGME, and PGMEA in a gas discharged through the upper portion of the distillation column 120 could be less than or equal to 100 ppm by weight, and leakage of the entrainer through the lower portion of the distillation column 120 could be prevented while the entrainer was not mixed with PGME in the presence of the catalyst in the distillation column 120.

Specifically, referring to FIG. 8, it was found that the entrainer did not leak through the lower portion of the distillation column 120 without leakage of PGME and PGMEA through the upper portion of the distillation column 120, and it was found that there was no section where the entrainer was mixed with PGME in the presence of the catalyst in the distillation column 120. (Referring to FIG. 8, there was no section where the entrainer, the catalyst, and PGME coexisted in the distillation column 120.)

In another embodiment, the pressure of the upper portion of the distillation column 120 may be changed. However, the pressure of the upper portion of the distillation column 120 may be adjusted in a range from -0.89 kg/cm²G to 0.5 kg/cm²G.

In a case where the pressure of the upper portion of the distillation column 120 was -0.625 kg/cm²G during operation, even when the temperature of the upper portion of the distillation column 120 was adjusted to about 48 °C, the temperature of the lower portion of the distillation column 120 was adjusted to about 115 °C, and the temperature of a certain separation stage above the inflow point where the liquid introduced from the reactor 110 into the distillation column 120 was adjusted to 52 °C, the concentration of PGME and PGMEA in the gas discharged through the upper portion of the distillation column 120 was less than or equal to 100 ppm by weight, the entrainer was not mixed with PGME in the presence of the catalyst in the distillation column 120 during operation, and the entrainer did not leak through the lower portion of the distillation column 120.

### [Example 3]

According to an embodiment, the distillation column 120 included 21 separation stages, and a mixture of water, acetic acid, PGME, PGMEA, and a catalyst was introduced from the reactor 110 into a middle point of the distillation column 120, and simultaneously, n-propyl acetate as an entrainer was introduced into the upper portion of the distillation column 120. P-toluenesulfonic acid (PTSA) having a boiling point of 140 °C at 20 mmHg was used as a catalyst.

The temperature of the upper portion of the distillation column 120 was determined by an azeotropic point of an entrainer and water and the pressure of the upper portion of the distillation column 120, and while the pressure of the upper portion of the distillation column 120 was set to the atmospheric pressure during operation, the temperature of the upper portion of the distillation column 120 was set to about 83 °C.

The temperature of the lower portion of the distillation column 120 was determined by a boiling point of a liquid-phase composition mixture discharged through the lower portion of the distillation column 120 and the pressure of the lower portion of the distillation column 120, and was set to about 135 °C.

The temperature of a certain separation stage above an inflow point where the liquid was introduced from the reactor 110 into the distillation column 120 was adjusted to about 116 °C so that the entrainer did not reach the inflow point, and the catalyst introduced into the inflow point was not mixed with the entrainer.

The temperature of the liquid introduced from the reactor 110 into the distillation column 120 was set to about 60 °C to about 135 °C as the liquid discharged through the lower portion of the distillation column 120 was introduced into the reactor 110 and then re-introduced into the distillation column 120.

In this regard, the temperature of the liquid introduced from the reactor 110 into the distillation column 120 may not be greater than the boiling temperature of the lower portion of the distillation column 120 to prevent large-scale evaporation of the catalyst above the inflow point.

In addition, when the temperature of the liquid introduced from the reactor 110 into the distillation column 120 is lower than 60 °C, energy may be overused by a reboiler, and thus, the temperature of the liquid introduced from the reactor 110 into the distillation column 120 may be greater than 60 °C.

As the reaction proceeds, the composition of the liquid discharged through the lower portion of the distillation column 120 changes, and thus, when the reaction ends, the maximum temperature may become the boiling temperature of the catalyst and PGMEA.

In other words, according to an embodiment, the temperature of the liquid introduced from the reactor 110 into the distillation column 120 may be greater than 60 °C and lower than the temperature of the lower portion of the distillation column 120.

The temperature of a certain separation stage above an inflow point where the liquid is introduced from the reactor 110 into the distillation column 120 may be determined depending on which stage is used as a reference, and the temperature may be between the temperature of the inflow point and the temperature of the top of the distillation column 120. The temperature of the inflow point is about 127 °C, but may be a boiling point at the corresponding pressure of a liquid composition formed at the inflow point, and thus, the temperature of the inflow point may vary as the reaction proceeds.

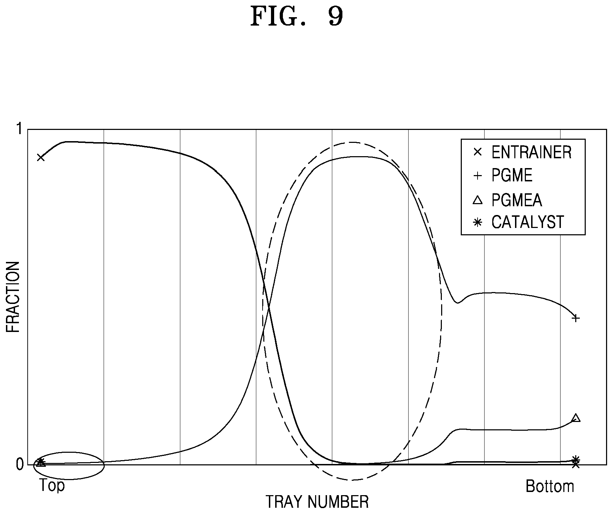

FIG. 9 is a composition distribution inside the distillation column 120 when n-propyl acetate was used as an entrainer. Referring to FIG. 9, through the above-described method, the concentration of acetic acid, PGME, and PGMEA in a gas discharged through the upper portion of the distillation column 120 could be lower than or equal to 100 ppm by weight, and leakage of the entrainer through the lower portion of the distillation column 120 could be prevented while the entrainer was not mixed with PGME in the presence of the catalyst in the distillation column 120.

Specifically, referring to FIG. 9, it was found that the entrainer did not leak through the lower portion of the distillation column 120 without leakage of PGME and PGMEA through the upper portion of the distillation column 120, and it was found that there was no section where the entrainer was mixed with PGME in the presence of the catalyst in the distillation column 120. (Referring to FIG. 9, there was no section where the entrainer, the catalyst, and PGME coexisted in the distillation column 120.)

In another embodiment, the pressure of the upper portion of the distillation column 120 may be changed. However, the pressure of the upper portion of the distillation column 120 may be adjusted in a range from -0.96 kg/cm²G to 0.5 kg/cm²G.

In a case where the pressure of the upper portion of the distillation column 120 was -0.625 kg/cm²G during operation, even when the temperature of the upper portion of the distillation column 120 was adjusted to about 60 °C, the temperature of the lower portion of the distillation column 120 was adjusted to about 116 °C, and the temperature of a certain separation stage above the inflow point where the liquid introduced from the reactor 110 into the distillation column 120 was adjusted to 90 °C, the concentration of PGME and PGMEA in the gas discharged through the upper portion of the distillation column 120 was less than or equal to 100 ppm by weight, the entrainer was not mixed with PGME in the presence of the catalyst in the distillation column 120 during operation, and the entrainer did not leak through the lower portion of the distillation column 120.

Isopropyl acetate, ethyl acetate, and n-propyl acetate according to an embodiment may be partially converted into isopropyl alcohol, ethyl alcohol, and n-propyl alcohol, respectively. The alcohol azeotropes with water, and thus, may be discharged through an upper portion of a distillation column.

A certain amount of the alcohol may be removed by moving a water layer in an oil-water separator to a separate distillation column for distillation or extraction. Unremoved alcohol may be refluxed to the distillation column together with an entrainer, but azeotropes with water together with the entrainer, and thus, may be discharged through the upper portion of the distillation column.

A certain amount of the alcohol mixed with the entrainer in an organic material layer in the oil-water separator may be removed in a separate distillation column. Since isopropyl acetate and isopropyl alcohol, ethyl acetate and ethyl alcohol, and n-propyl acetate and n-propyl alcohol azeotrope under the atmospheric pressure, respectively, the separate distillation column may remove a certain amount of the alcohol by reducing the composition of the alcohol to be azeotroped by operating at high pressure.

### [Comparative Example]

According to Comparative Example, a distillation column included 21 separation stages, and a mixture of water, acetic acid, PGME, PGMEA, and a catalyst was introduced from a reactor into a middle point of the distillation column, and simultaneously, toluene as an entrainer was introduced into an upper portion of the distillation column. When the pressure of the upper portion of the distillation column was -0.625 kg/cm²G during operation, the temperature of the upper portion of the distillation column was set to 62 °C, and the temperature of a lower portion of the distillation column was set to 115 °C.

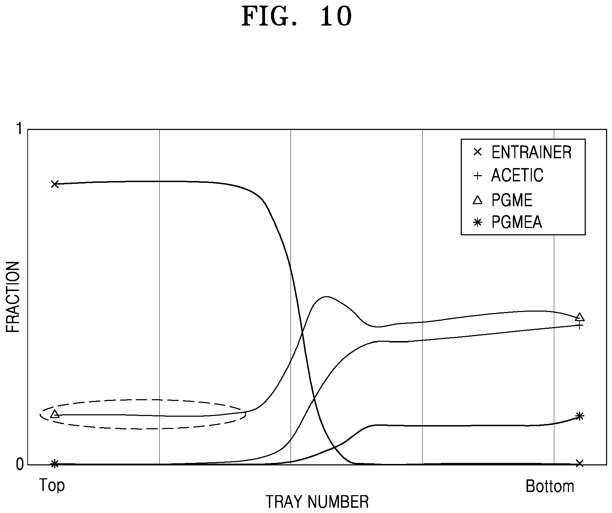

FIG. 10 is a composition distribution inside the distillation column when toluene was used as an entrainer. Referring to FIG. 10, when toluene was used as an entrainer, the concentration of acetic acid and PGMEA in a gas discharged through the upper portion of the distillation column was less than or equal to 100 ppm by weight, but the concentration of PGME was about 14 % by weight, and a large amount of PGME was leaked through the upper portion of the distillation column.

This was because water-PGME-toluene azeotroped at the lowest temperature, and since PGME was a water-soluble solvent, a large amount of PGME was included in a water layer of a PGME-condensed liquid phase and lost. In a case where toluene was used as an entrainer, even when the temperature of the area above a point where the liquid was introduced from the reactor into the distillation column was adjusted by any method, leakage of PGME through the upper portion of the distillation column could not be avoided.

In other words, when isopropyl acetate, ethyl acetate, and n-propyl acetate are each used as an entrainer as in Examples, leakage of PGME and PGMEA through the upper portion of the distillation column 120 may be prevented, leakage of the entrainer through the lower portion of the distillation column 120 may be prevented, and simultaneously, a section in which the entrainer is mixed with PGME in the presence of a catalyst in the distillation column 120 may be removed, whereas when an entrainer such as toluene that azeotropes with PGME or an entrainer that azeotropes with PGMEA is used as in Comparative Example, leakage of PGME and PGMEA through the upper portion of the distillation column may not be prevented.

As such, a method of removing water from a mixture of PGME, PGMEA, and water according to an embodiment may increase a reaction rate for the production of PGMEA and simultaneously increase the purity of PGMEA by removing, from a reactant and a reaction product, water produced as a by-product in the production of PGMEA used as an environmentally friendly solvent.

The method of removing water from the mixture of PGME, PGMEA, and water according to an embodiment has the following effects.

The method of removing water from the mixture of PGME, PGMEA, and water according to an embodiment has an advantage in that a reaction rate for the production of PGMEA may be increased by removing, from a reactant and a reaction product, water produced as a by-product in the production of PGMEA used as an environmentally friendly solvent.

In addition, the method of removing water from the mixture of PGME, PGMEA, and water according to an embodiment has an advantage in that the purity of PGMEA may be increased by removing, from a reactant and a reaction product, water produced as a by-product in the production of PGMEA used as an environmentally friendly solvent.

Furthermore, the method of removing water from the mixture of PGME, PGMEA, and water according to an embodiment has an advantage in that loss of a reactant and a reaction product may be minimized by removing, from the reactant and the reaction product, water produced as a by-product in the production of PGMEA used as an environmentally friendly solvent, and accordingly, PGMEA which is an environmentally friendly solvent used as a paint solvent, an electronic material solvent, or the like may be economically produced.

As such, the disclosure has been described with reference to embodiments shown in the drawings, but these are only exemplary, and it will be understood by those of ordinary skill in the art that various changes in form and details may be made therein without departing from the spirit and scope of the present invention as defined by the following claims.

## Claims

1. A method of removing water from a mixture of PGME, PGMEA, and water, the method comprising:
a reactor introduction step of introducing PGME and acetic acid into a reactor;
a distillation column introduction step of introducing a liquid in the reactor into a distillation column;
an entrainer introduction step of introducing an entrainer into an upper portion of the distillation column; and
a condensation step of condensing, in a condenser, a mixture of water and the entrainer, which evaporates from the upper portion of the distillation column.

2. The method of claim 1, wherein the entrainer comprises at least one of isopropyl acetate, ethyl acetate, and n-propyl acetate.

3. The method of claim 1, further comprising:
a reflux step of separating a liquid phase condensed in the condenser into an organic material layer and a water layer, and refluxing the organic material layer to the distillation column; and
a discharge step of separating a liquid phase condensed in the condenser into an organic material layer and a water layer, and discharging the water layer.

4. The method of claim 1, wherein a catalyst to promote a reaction between the PGME and the acetic acid is mixed with the liquid introduced into the reactor and introduced into the distillation column.

5. The method of claim 4, wherein the catalyst comprises a material having a boiling temperature of 220 °C or higher under the atmospheric pressure or having a boiling temperature of 140 °C or higher at 20 mmHg.

6. The method of claim 1, wherein a solid catalyst to promote a reaction between the PGME and the acetic acid are filled in the distillation column, and
the solid catalyst is filled under an inflow point where the liquid in the reactor is introduced into the distillation column.

7. The method of claim 1, further comprising a re-introduction step of re-introducing the liquid discharged through a lower portion of the distillation column into the reactor.

8. The method of claim 1, wherein the number of separation stages of the distillation column is 21 or more.

9. The method of claim 1, wherein an inflow point where the liquid in the reactor is introduced into the distillation column is a point apart from the upper portion of the distillation column by 8 separation stages, or is lower than the point apart from the upper portion of the distillation column by 8 separation stages.

10. The method of claim 1, wherein an inflow point where the liquid in the reactor is introduced into the distillation column is a point apart from a lower portion of the distillation column by 2 separation stages, or is higher than the point apart from the lower portion of the distillation column by 2 separation stages.

11. The method of claim 1, wherein the reactor and the distillation column are integrally formed.

12. The method of claim 1, wherein a temperature of the upper portion of the distillation column is greater than or equal to an azeotropic point of the mixture of the entrainer and the water, and
a temperature of a lower portion of the distillation column is a boiling point of the mixture discharged through the lower portion of the distillation column.

13. The method of claim 2, wherein, when the entrainer is isopropyl acetate, a pressure of the upper portion of the distillation column is -0.925 kg/cm²G to 0.5 kg/cm²G,
when the entrainer is ethyl acetate, a pressure of the upper portion of the distillation column is -0.89 kg/cm²G to 0.5 kg/cm²G, and
when the entrainer is n-propyl acetate, a pressure of the upper portion of the distillation column is -0.96 kg/cm²G to 0.5 kg/cm²G.

14. The method of claim 1, wherein an inflow point where the liquid in the reactor is introduced into the distillation column is 90 °C to 140 °C when a pressure of the upper portion of the distillation column is the atmospheric pressure.

15. The method of claim 1, wherein the distillation column comprises at least one of a tray, random packing, and structured packing.
